# EUROPEAN PATENT SPECIFICATION

(11) **EP 0 275 298 B1**
(45) Date of publication and mention of the grant of the patent: **01.03.1995**
(21) Application number: 87905100.1
(22) Date of filing: 22.07.1987
(51) Int. Cl.: C12Q 1/68, C07H 21/00

(54) **DMD PROBES**
DMD-SONDEN
SONDES DMD

(30) Priority: 25.07.1986 US 890694
(43) Date of publication of application: 27.07.1988
(73) Proprietor: The Children's Medical Center Corporation, Boston, Massachusetts 02115 (US)
(72) Inventor: KUNKEL, Louis, M., Hyde Park, MA 02136 (US); MONACO, Anthony, Boston, MA 02115 (US)
(74) Representative: Deans, Michael John Percy
(86) International application number: US8701749
(87) International publication number: WO8800979

(56) References cited:
- NUCLEIC ACIDS RESEARCH, vol. 13, no. 1, 11 January 1985, Oxford (GB); K.E. DAVIES et al., p. 155#
- CELL, vol. 47, 21 November 1986, Cambridge, MA (US); G.J.B. VAN OMNEN et al.,pp. 499, 501#
- NATURE, vol. 316, no. 6031, 29 August 1985, London (GB); A.P. MONACO et al., pp. 842, 844#
- AMERICAN JOURNAL OF HUMAN GENETICS, vol. 37, no. 2, March 1985, Chicago, IL (US); B. de MARTINVILLE et al., pp. 235, 239#
- PROCEEDINGS OF THE NATL. ACADEMY OF SCIENCES USA, vol. 82, July 1985; L. KUNKEL, pp. 4778-4782#
- NUCLEIC ACIDS RESEARCH, vol. 14, no. 17, 11 September 1986, Oxford (GB); J. KOCH et al., p. 7133#
- CHEMICAL ABSTRACTS, vol. 103, no. 1, 08 July 1985, Columbus, OH (US); L.M. KUNKEL et al., p. 151, no. 1538p#
- NATURE, vol. 318, no. 6047, 19 December 1985, London (GB); P.N. RAY et al., pp. 672, 674#
- THE LANCET, vol. I, no. 8430, 23 march 1985, Boston, MA (US); E. BAKKER et al., pp. 655-658#
- CHEMICAL ABSTRACTS, vol. 105, no. 23, 08 December 1986, Columbus, OH (US); A. SPEER et al., p. 149, no. 204055t#

## Description

### Background of the Invention

This invention relates to the detection of hereditary disease.

Duchenne muscular dystrophy (DMD) is an X-linked recessive genetic disorder which affects about 1 in 3,300 males and for which the biochemical defect is as yet unknown. Traits associated with DMD (DMD phenotype) are well known and may include elevated creatine phosphokinase levels in serum, delayed development of motor function, and muscle weakness characterized by the replacement of muscle fiber with adipose and fibrose tissue accompanied by a marked variation in muscle size. Until recently carrier identification in DMD families generally was accomplished by detecting elevated levels of creatine phosphokinase in serum.

Restriction fragment length polymorphisms (RFLP's) occur when the DNA sequence in the genone varies such that digestion with a restriction endonuclease will sometimes result in a set of restriction fragments of one size array, and sometimes in fragments of one or more different size arrays. The differences are due to the presence or absence in the DNA sequence of sites recognized by the endonuclease; a sequence in which the site is present will be cut by the endonuclease giving one array, and a sequence in which the site is not present will fail to be cut by the endonuclease, giving another array.

It has been shown that RFLP's which are located near a gene associated with a hereditary disease can be used to determine the likelihood of a particular person having a defective gene and thus having the disease. Bakker et al., The Lancet, March 23, 1985, at 655 describes "eleven RFLP-markers... on the short arm of the X chromosome [that] are useful in the diagnosis of DMD since they bridge the Duchenne locus at genetic distances varying between 3 and 20 centimorgans."

### Summary of the Invention

In one aspect, the invention features a human DMD probe comprising a single-stranded nucleic acid sequence capable of hybridizing to a first region of DNA on a human X chromosome, said human X chromosome having a first point designated as the deletion break point at Xp21.3 and a second point designated as the translocation break point x;11, said first region of DNA being positioned between said first point and said second point, said nucleic acid sequence being incapable of hybridizing to a second region of DNA between said first point and a terminus of said X chromosome, and being incapable of hybridizing to a third region of DNA between said second point and the centromere of said X chromosome, said probe sequence capable of hybridizing being all or a fragment of the sequence of pERT87 deposited under ATCC Accession No: 67162.

These probes are particularly suitable for detecting RFLPs that are closely associated (linked) with mutations in the DMD gene causing a DMD phenotype. By closely associated, it is meant that the likelihood of a genetic recombination event occurring between the sites of an RFLP and a DMD mutation is very low (less than 5%). Since the probes of the invention are within or very close to the DMD gene they are closely associated with DMD mutations. The advantage of such probes is that one can use them to predict, with a high degree of confidence, the probability that an individual (within a family with a history of DMD) having a particular RFLP phenotype will also have a DMD mutation.

The probe of the invention can be used for analyzing a sample of genomic DNA of a human subject for the presence of a mutant.DMD gene.

The method of the invention can be used for prenatal screening as well as in the detection of heterozygotic individuals carrying DMD mutations but exhibiting no symptoms.

Other features and advantages of the invention will be apparent from the following description of the preferred embodiments thereof, and from the claims.

### Description of the Preferred Embodiment

The drawings will first briefly be described.

### Drawings

Fig. 1 is a standard ISCN (1981) ideogram of the p terminal end of the human X chromosome showing the approximate positions of cloned DNA fragments and specific deletion and translocation break points;
Fig. 2 is a diagrammatic representation of 220kb of cloned DNA from the human Xp21 chromosome, showing regions deleted in some DMD human males;
Fig. 3 is the DNA sequence of two parts of the DMD gene;
Fig. 4 is a diagrammatic representation of 220kb of cloned DNA showing the location and size of cloned DNA isolated from librares of human chromosomal DNA; and
Fig. 5 is a schematic representation of the genotype of each family member, circles represent females, squares represent males, darkened areas indicate the presence of a DMD mutant gene.

### Structure

Probes of the invention contain nucleic acid homologous to DNA of the DMD gene or to DNA from a region of DNA close to the DMD gene. A description of the DNA region containing segments to which the probes are homologous follows.

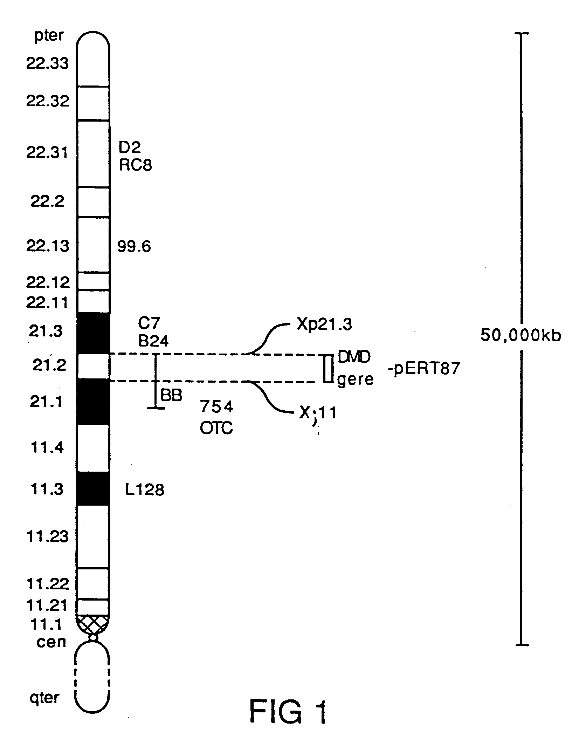

Referring to Fig. 1, the approximate positions of previously cloned regions of the human X chromosome (D2, RC8, 99.6, 754, OTC C7, B24, and L128) are shown relative to a standard (ISCN 1981) ideogram of the short arm (p) of the human X chromosome. Also shown is the region of DNA deletion in the X chromosome of a male patient (BB) suffering from three X-linked disorders, including DMD; the deletion is described by Francke et al., 37 Am. J. Hum. Genet. 250 (1985), and the cell line is available from Human Genetic Mutant Cell Repository in Camden, N.J. (cell line repository number GM7947A). The deletion is defined by two break points in the DNA, Xp21.3 and Xp21.1. Also marked is a break point X;11 (p. 21.1; a 13.5), which is attributable to the balanced reciprocal translocation described by Greenstein et al., 27 Cytog. Cell Genet. 268 (1980) (cell line available from Human Genetic Mutant Cell Repository in Camden, N.J., cell line repository identification number GM1695).

Break points Xp21.3 and X;11 define approximately the boundaries of the DMD gene, which lies within the Xp21.2 chromosome band region.

In order to isolate the probes of the invention, a library of DNA from around the DMD gene area was constructed by enriching for this DNA using differential hybridization. The procedure is described in detail by Kunkel et al., 82 PNAS 4778 (1985). Accordingly, 250 »g of DNA from the male patient BB was isolated, sheared by sonication to a mean size of 1,000 base pairs (bp), and hybridized to 1.25 »g of Mbo-1 cleaved DNA from a 49, XXXXY lymphoid cell line (GM1202), as described by Palmer et al., 37 Cell 171 (1984). The DNAs then were heated 100°C for 5 minutes, cooled on ice, and added to a final reaction volume of 2.5 ml of 7% phenol. The mixture was shaken intermittently for 37 h over a 5 day period (Kohne et al. 1977, Biochemistry 16:5329). After chloroform extraction and dialysis to remove salts, the hybridized DNA was ethanol precipitated. These conditions allow Mbo-1 fragments from the 49 XXXXY DNA, with no complementary sequence in the deleted DNA region of the BB male, to self-hybridize and thus be cloned as Mbo-1 fragments. 5 »g of the precipitated DNA was ligated to 0.1 »g BamH1-cleaved dephosphorylated pBR322 and transformed into E. coli strain MC1061. (The BamH1 ends are compatible in ligation with the Mbo-1 ends of the chromosomal DNA.) 3,000 colonies were isolated.

As described by Kunkel, supra, the isolated colonies were screened using standard techniques to determine which one contained DNA that hybridized with DNA from normal individuals but not with DNA from the male (BB). One clone, pERT87, hybridized to a 1.1 kb HindIII fragment of normal human DNA, but not to BB DNA. The intensity of hybridization was dependent upon the X chromosome content of the DNA being probed. Hybridization was also detected in a rodent-human hybrid cell line having an intact human X chromosome, but not with a cell line having DNA only from the human chromosome region Xp11.3 to Xqter. Thus, the cloned region in pERT87 is on the short arm (p) of the X chromosome. Three other clones gave similar results, although each clone hybridizes to a different HindIII fragment.

The clones were analyzed for their ability to hybridize with DNA from a variety or cell lines having deltions or translocations of the human X chromosome. pERT87 was localized to DNA within the Xp21-Xp22 region and pERT84 to DNA within the region Xp11.3-Xp21. The location of pERT87 was determined by its lack of hybridization to the region Xp21-Xter (using cell line X;11) and to the region p21-pter (using a cell line with a break point at Xp21.3, Fryns et al. 22 Clin. Genet. 76, 1982), and by its hybridization to the region Xp22-Xqter (using a cell line described by Mahandas et al., 1979, PNAS 76:5779).

As described in Monaco et al., 316 Nature 842 (1985), pERT87 was used to probe the DNA of 57 unrelated DMD males. pERT87 did not hybridize to DNA from five of the males indicating that they had deletions of DNA around the cloned DNA in pERT87. Assuming that the DMD phenotype in these five males is a result of the deletions being within the DMD gene, this result provides evidence that pERT87 contains DNA homologous to a region of DNA within the DMD gene.

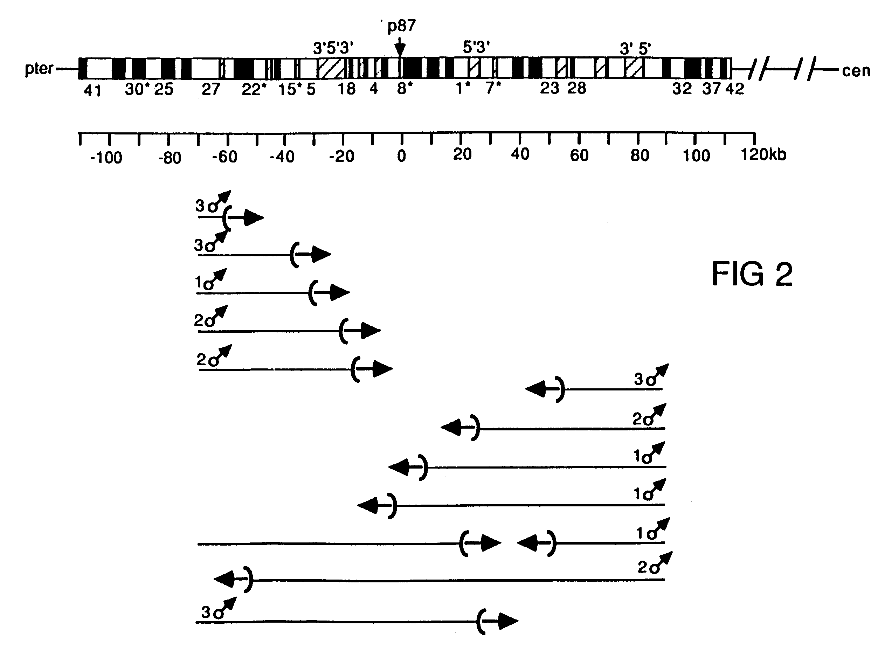

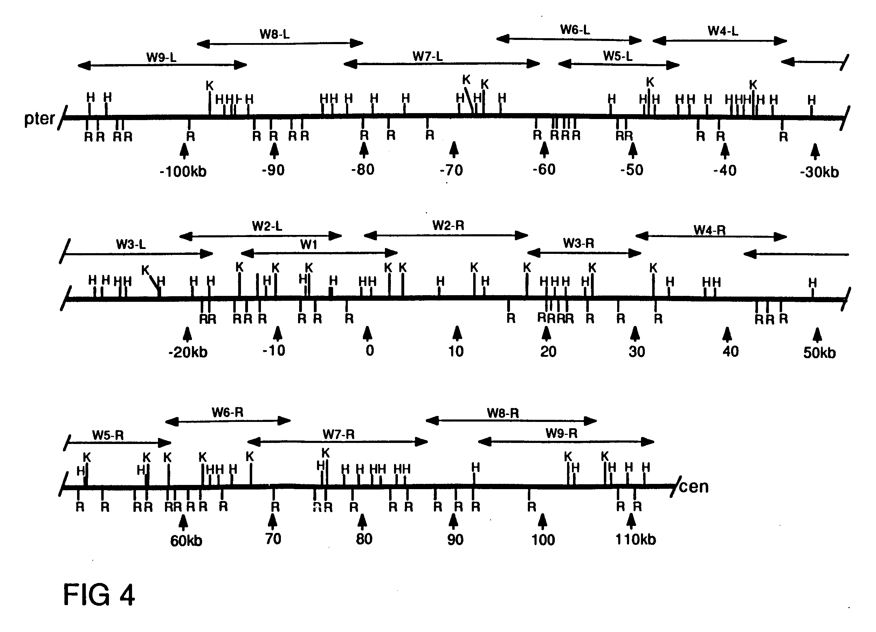

In order to isolate more DNA from the DMD region, two human genomic libraries were screened with pERT87 by the procedure described in Monaco, supra; one was constructed in Charon 35 (Loenan et al., 1983, Gene 26:171) and one in EMBL-3 (Frischauf et al., 1983, J. Molec. Biol. 170:827. Using standard techniques chromosomal "walks" were performed in both directions from the region homologous to pERT87 using pERT87 as a probe for the above libraries. Five bacteriophage clones, including W1 and W2-R in Fig. 4, were isolated, and small unique-sequence subclones, in pBR322, puC18 or Blue-Scribe (Stratagene), constructed. These subclones were restriction mapped and small fragments free of human repetitive elements identified. Unique sequence subclones near the extreme ends of the human inserts in these clones were used as probes for the next walking step in the same libraries. Three of these subclones, pERT87-18, -8, and -1 contained DNA absent from the above five DMD boys with genomic deletions. These subclones span an area of 38 kb. Further chromosome walks in the EMBL-3 library extended the cloned area to 220 kb (shown in Figs. 2 and 4). These clones include pERT87-15, -14, and -27.

To determine the positions of these cloned regions relative to the DMD gene, chromosomal DNA was isolated from 53 boys with DMD or the related disease, Becker muscular dystrophy--the gene for which is also located within band Xp21--who have deleted areas of DNA in their X chromosome. This DNA was tested, using standard Southern blot analysis (see below), against the subclones in order to determine the break points of the DNA deletions in the 53 boys relative to the cloned DNA. This was simply done by firstly ordering the subclones as shown in Fig. 4, and then determining which subclones contained DNA present in the deleted chromosomes (and thus are outside the break point) and those which contained DNA are not present in the deleted chromosome (and thus have DNA within the break point). Subclones positioned between these two types of subclones, and having DNA present in the deleted chromosomes, must have DNA in which the deletion break point occurred. Once localized to a region within the cloned DNA, the break points were further localized within 100=1,000 bp using restriction enzyme analysis. The results of these experiments are summarized in Fig. 2.

Referring to Fig. 2, a schematic drawing of 220 kb of contiguous human DNA is presented with a kb scale. The darkened blocks represent regions of repeated sequences, cross hatched boxes represent moderately repeated sequences, and open boxes represent unique sequences. Numbers below these blocks represent pERT87 subclones. Line sequences are represented by an L, as described in Singer 28 Cell 433 (1981). As shown in Fig. 2, fourteen deletions were found to break at the left side of the cloned DNA and extend in a rightward direction (see arrows) towards the centromere. Nine deletions were found that extend in the other direction. The majority of the deletions have independent break points, but for simplicity the deletions have been schematically presented together in Fig. 2. Since the deletion breaks, which give rise to a DMD or BMD phenotype, occur within the pERT87 region and extend in both directions, this analysis demonstrates that pERT87 and the subclones are clearly within the DMD gene.

The profile in Fig. 2 indicates that the DMD gene is located on a large segment of DNA. The fact that large deletions resulting from mutations in the gene yield a DMD phenotype similar to other DMD boys who have not been demonstrated to have deletions indicates that the gene product can be either completely missing or abberant and still yield a similar clinical picture. The gene responsible from analysis of various mutations causing DMD lies on a very large segment of X chromosome DNA, and is estimated at approximately 2,000 kb (Fig. 1).

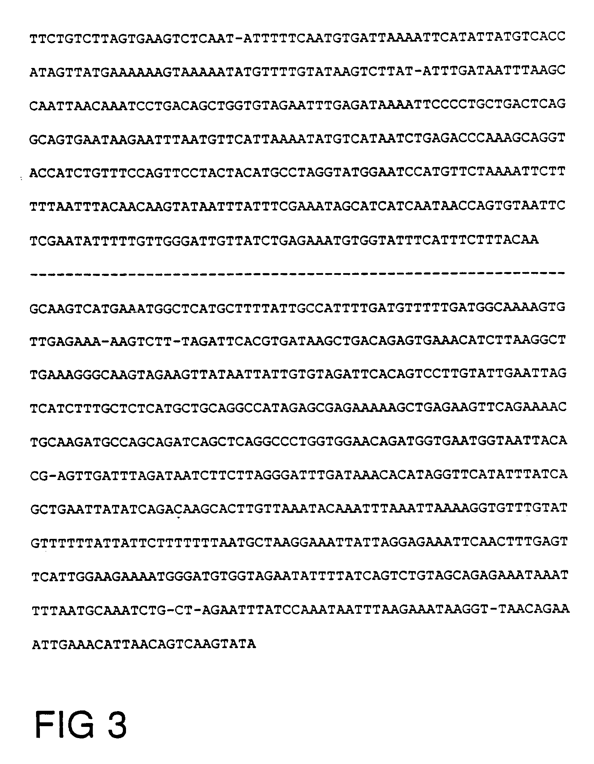

Referring to Fig. 3, the DNA sequence of several subclones are determined by standard procedures, using dideoxy analysis. Representative sequences from pERT87-4 (upper sequence) and -25 (lower sequence) are presented. Probes suitable for the invention can be readily synthesized from any parts of the sequences, and such probes can also be used for further chromosome walking.

Northern analysis, using standard procedures, of RNA from a variety of human fetal and adult tissues revealed a single 18 kb mRNA from a total RNA or polyA-selected RNA of fetal skeletal muscle which hybridizes to pERT87-25. No transcripts were detected in adult skeletal muscle tissue. This 18 kb transcript is presumably the mRNA, coding for the product of the DMD gene. It is possible to isolate this mRNA, using standard hybridization techniques, and the probes of the invention, and to prepare a cDNA library from it. The clones in this cDNA library will be suitable as probes of the invention, and for isolating other clones by chromosome walking in human genomic DNA library by standard techniques towards intronic DNA in the DMD gene and to the X;11 and Xp21.3 break points.

For example, an oligo (dT) primed cDNA library was constructed in the phage vector lambda gt11 using a poly A-selected RNA sample from human fetal skeletal muscle. Approximately 1.5 million phage were screened using both pERT87-25 and pERT87-4 as hybridization probes, and 20 phage positives were isolated. All 20 hybridized only with pERT87-25. Of the 20 phage positives isolated with pERT87-25, 5 different cDNA clones with different sized inserts ranging from 1.0 to 2.9 kb were characterized further. One cDNA clone of 2.0 kb gave a repetitive pattern of hybridization to genomic DNA. The remaining cDNA clones hybridized to human genomic DNA with Hind III restriction fragments that map to the region defined in this application between Xp21.3 and X;11.

Analysis of these cDNA clones demonstrates that the average intron size is 16 kb over the 220 kb of cloned DNA. If this is true for the remainder of the 18 kb coding RNA, the genomic gene for DMD would cover 2,000 kb.

In order to test whether any particular cloned DNA is suitable as a probe of the invention, the clones can be hybridized, using standard methods, to cell lines having the Xp21.3 and X;11 mutations and to mRNA from human cell lines. Suitable probes will have homology to DNA from within the Xp21.3 -X;11 region or within the mRNA. Referring to Fig. 1, suitable probes have homology to normal human cell lines but at least part of their DNA will not have homology to both DNA from cell line GM7947A and the nontranslocated DNA of the cell line GM1695. A further test entails a cytological study to determine whether the clone hybridizes to the Xp21.2 chromosomal band, and thus would be suitable.

### Use

The probes of the invention are useful in detecting RFLP's that are closely linked with a mutated DMD gene that gives rise to the DMD phenotype. Detection of such RFLP's allows diagnosis bath of women who are carriers of a mutated DMD gene and unborn children who have a mutated DMD gene.

To detect polymorphisms in the length of restriction fragments, an analytical method for fractioning double-stranded DNA molecules on the basis of size is required. The most commonly used technique for achieving such a fractionation is agarose gel electrophoresis. The principle for this method is that DNA molecules migrate through the gel as though it were a sieve that retards the movement of the largest molecules of the greatest extent and the movement of the smallest molecules to the least extent.

The DNA fragments fractionated by agarose gel electrophoresis can be visualized directly by a staining procedure if the number of fragments included in the pattern is small. However, most genomes, including the human genome, contain far too many DNA sequences to produce a simple pattern of restriction fragments. For example, the human genome is digested into approximately 1,000,000 different DNA fragments by EcoRI. In order to visualize a small subset of these fragments the Southern hybridization procedure can be applied.

The purpose of the Southern transfer procedure (also referred to as blotting) is to transfer DNA fractionated by agarose gel electrophoresis onto a nitrocellulose fiber paper while retaining the relative positions of DNA fragments resulting from the fractionation procedure. The methodology used to accomplish the transfer from agarose gel to nitrocellulose is to draw the DNA from the gel into the nitrocellulose paper by capilliary action.

Nucleic acid hybridization depends on the principle that two single-stranded nucleic acid molecules that have complementary base sequences will reform the thermodynamically favored double-stranded structure if they are mixed in solution under the proper conditions. The double-stranded structure will be formed between two complementary single-stranded nucleic acids even if one is immobilized on a nitrocellulose filter. In the Southern hybridization procedures the latter situation occurs. The DNA of the individual to be tested is digested with a restriction endonuclease, fractionated by agarose gel electrophoresis, converted to the single-stranded form, end transferred to nitrocellulose paper, making it available for reannealing to the hybridization probe.

To visualize a particular DNA sequence in the Southern hybridization procedure, a labeled DNA molecule or hybridization probe is reacted to the fractionated DNA bound to the nitrocellulose filter. The areas on the filter that carry DNA sequences complementary to the labeled DNA probe become radioactively labeled themselves as a consequence of the reannealing reaction. The areas of the filter that exhibit such radioactive labeling are visualized by autoradiography.

Using standard techniques, the subclones pERT87-8, and pERT87-15 were tested for their ability to detect human DNA RFLP's by hybridizing to nitrocellulose filters containing immobilized DNA samples from four unrelated females that had been cleaved with 24 different restriction enzymes. The frequencies of the RFLP's found in the human population were estimated by examining DNA obtained from 37 unrelated persons. The results of the search are displayed in Table 1, below. Seven potential polymorphic restriction enzyme sites were identified as individual variations in the pattern of restriction fragments observed. Two of the subclones, pERT87-1 and pERT87-8, each detected putative RFLP's of two different enzymes, while one probe, pERT87-15, detected RFLP's of three different enzymes.

**Table 1**

| RFLP Detecting Subclones of pERT87 (DXS 164) | | | | | | |
|---|---|---|---|---|---|---|
| 1. | Subclone | Enzyme | Restriction Fragment Length Polymorphisms | | | |
| | | | Allele Sizes (kb) | | Allele Frequency | |
| | | | p* | q** | p | q |
| 2. | pERT87-1 | BstNI | 3.1 | 2.5/0.6 | 0.63 | 0.37 |
| 3. | | XmnI | 8.7 | 7.5 | 0.66 | 0.34 |
| 4. | pERT87-8 | BstXI | 4.4. | 2.2 | 0.6 | 0.4 |
| 5. | | TaqI | 2.7/1.1 | 3.8 | 0.71 | 0.29 |
| 6. | pERT87-15 | BamHI | 7.1/2.3 | 9.4 | 0.62 | 0.38 |
| 7. | | TaqI | 3.1 | 3.3 | 0.67 | 0.33 |
| 8. | | XmnI | 1.6/1.2 | 2.8 | 0.68 | 0.32 |

| | | | | | | |
|---|---|---|---|---|---|---|
| * common allele | | | | | | |
| ** rare allele | | | | | | |

The next step was to screen the RFLP's clones for linkage to the DMD phenotype. A RFLP is linked to the DMD gene within a particular pedigree (family) if within the pedigree, persons whose DNA contains a mutant DMD gene giving rise to the DMD phenotype have a polymorphic distribution different from those persons within the pedigree whose DNA does not contain a mutant DMD gene. The RFLP screening was performed on pedigrees known to harbor a mutant DMD gene.

The first step was to obtain DNA from a subject. In the method described below, peripheral blood lymphocytes are used; other possible sources of DNA are amniotic fluid, chorionic villus, and fetal trophoblasts.

Genomic DNA was isolated from cell nuclei of whole blood leukocytes as described by Aldridge et al., 36 Am. J. Hum. Gen. 546 (1984). The harvested DNA was then digested with the selected restriction enzyme under conditions specified by the enzyme's manufacturer. The digested DNA was separated by electrophoresis on horizontal 0.7% agrose gel and transferred to nitrocellullose as described by Southern, 98 J. Mol. Bio. 503 (1975). The clones ³²P-radiolabelled using T4 DNA polymerase and radiolabelled fragments recovered from low melting point agarose gels, as described by Aldridge, supra. Prehybridization of nitrocellulose filters is performed as bescribed by Aldridge, supra and hybridization was at 45°C in 50% deionized formamide. 4 X SSC (1 X SSC = 0.15 M NaCl, 0.015 M Na citrate), 0.05 M sodium phosphate buffer (pH 6.8), 8% dextran sulphate, 10 X Denhardt's, 100»g/ml transfer RNA, 25 »g/ml sheared salmon sperm DNA, 0.1% SDS, 1mM EDTA and 10⁶ c.p.m. ml⁻¹ of radiolabelled DNA. Filters were washed in 0.1% SDS and 0.1 X SSC several times at room temperature and ten for 1 h at 55°C. Autoradiography was at -70°C with a DuPont intensifying screen for 1-5 days.

The analysis showed linkage between the RFLPs of Table 1 and defective DMD genes with certain families.

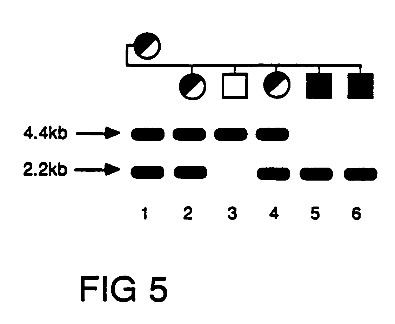

An example of such linkage is presented in Figure 5. In the example, DNA was isolated from individuals within a family in which some people had a mutated DMD gene (indicated by darkened symbols) the DNA was cleaved with BstXI, and probed with pERT87-8. Electrophoresis, blotting, and hybridization were as described above. On BstXI digestion of genomic DNA, pERT87-8 hybridizes to 4.4 and 2.2 kb BstXI fragments in females heterozygous for the RFLP. The carrier mother exhibits the heterozygous pattern of the 4.4/2.2 kb BstXI alleles, as do her two carrier daughters (lanes 2,4). The lower allele (2.2 kb) is present in both affected DMD sons (lanes 5,6) while only the upper allele (4.4 kb) is present in the unaffected son (lane 3). The father of this family has a 4.4 kb BstXI hybridizing fragment (data not shown). The female individuals in lanes 2 and 4 were suspected of being carriers of the DMD trait based on elevated CPK values. Fragment sizes were calculated by comparison with ³²P-end-labelled HindIII-cleaved λ phage DNA. Thus, the mutated DMD gene is linked to the BstXI RFLP in this family, and individuals with 2.2 kb fragment also have a mutated DMD gene.

Linkage between each RFLP listed in Table 1 and a defective DMD gene does not occur in every pedigree. Accordingly, a combination of probes, detecting a plurality of RFLP's, is preferably used when examining DNA of an individual. Examining a combination of RFLP's listed on lines 2, 3, 5, 6, and 7 (of Table 1) with the appropriate probes was found to be informative in 25 of 28 (89%) pedigrees in which members were known to have a mutated DMD gene. It is also possible to use probes of the invention for presymptomatic screening of humans without a DMD phenotype for deletions (of greater than 100 bps) in the DMD gene.

Other embodiments are within the following claims.

### Deposit

An E. coli strain having a plasmid containing the pERT87 and cloned DNA deposited on July 24, 1986 with the American Type Culture Collection, Rockville, Maryland, and assigned ATCC accession number 67162. Applicants' assignee, Children's Medical Center Corporation, represents that the ATCC is a depository affording permanence of the deposit and ready accessibility thereto by the public if a patent is granted. All restrictions on the availability to the public of the material so deposited will be irrevocably removed upon the granting of a patent. The material will be available during the pendency of the patent application to one determined by the Commissioner to be entitled thereto under 37 CFR 1.14 and 35 USC 112. The deposited material will be maintained with all the care necessary to keep it viable and uncontaminated for a period of at least five years after the most recent request for the furnishing of a sample of the deposited microorganism, and in any case, for a period of at least thirty (30) years after the date of deposit or for the enforceable life of the patent, whichever period is longer. Applicants' assignee acknowledges its duty to replace the deposit should the depository be unable to furnish a sample when requested due to the condition of the deposit.

## Claims

1. A human Duchenne Muscular Dystrophy (DMD) probe comprising a single-stranded nucleic acid sequence capable of hybridizing to a first region of DNA on a human X chromosome, said human X chromosome having a first point designated as the deletion break point at Xp21.3 and a second point designated as the translocation break point x;11, said first region of DNA being positioned between said first point and said second point, said nucleic acid sequence being incapable of hybridizing to a second region of DNA between said first point and a terminus of said X chromosome, and being incapable of hybridizing to a third region of DNA between said second point and the centromere of said X chromosome, said probe sequence capable of hybridizing being all or a fragment of the sequence of pERT87 deposited under ATCC Accession No: 67162.

2. A human DMD probe comprising at least one of the sequences 
TTCTGTCTTAGTGAAGTCTCAAT-ATTTTTCAATGTGATTAAAATTCATATTATGTCACC
ATAGTTATGAAAAAAGTAAAAATATGTTTTGTATAAGTCTTAT-ATTTGATAATTTAAGC
CAATTAACAAATCCTGACAGCTGGTGTAGAATTTGAGATAAAATTCCCCTGCTGACTCAG
GCAGTGAATAAGAATTTAATGTTCATTAAAATATGTCATAATCTGAGACCCAAAGCAGGT
ACCATCTGTTTCCAGTTCCTACTACATGCCTAGGTATGGAATCCATGTTCTAAAATTCTT
TTTAATTTACAACAAGTATAATTTATTTCGAAATAGCATCATCAATAACCAGTGTAATTC
TCGAATATTTTTGTTGGGATTGTTATCTGAGAAATGTGGTATTTCATTTCTTTACAA
 and

GCAAGTCATGAAATGGCTCATGCTTTTATTGCCATTTTGATGTTTTTGATGGCAAAAGTG
TTGAGAAA-AAGTCTT-TAGATTCACGTGATAAGCTGACAGAGTGAAACATCTTAAGGCT
TGAAAGGGCAAGTAGAAGTTATAATTATTGTGTAGATTCACAGTCCTTGTATTGAATTAG
TCATCTTTGCTCTCATGCTGCAGGCCATAGAGCGAGAAAAAGCTGAGAAGTTCAGAAAAC
TGCAAGATGCCAGCAGATCAGCTCAGGCCCTGGTGGAACAGATGGTGAATGGTAATTACA
CG-AGTTGATTTAGATAATCTTCTTAGGGATTTGATAAACACATAGGTTCATATTTATCA
GCTGAATTATATCAGACAAGCACTTGTTAAATACAAATTTAAATTAAAAGGTGTTTGTAT
GTTTTTATTATTCTTTTTTAATGCTAAGGAAATTATTAGGAGAAATTCAACTTTGAGT
TCATTGGAAGAAAATGGGATGTGGTAGAATATTTATCAGTCTGTAGCAGAGAAATAAAT
TTTAATGCAAATCTG-CT-AGAATTTATCCAAATAATTTAAGAAATAAGGT-TAACAGAA
ATTGAAACATTAACAGTCAAGTATA

3. A human DMD probe comprising the sequences 
TTCTGTCTTAGTGAAGTCTCAAT-ATTTTTCAATGTGATTAAAATTCATATTATGTCACC

ATAGTTATGAAAAAAGTAAAAATATGTTTTGTATAAGTCTTAT-ATTTGATAATTTAAGC

CAATTAACAAATCCTGACAGCTGGTGTAGAATTTGAGATAAAATTCCCCTGCTGACTCAG

GCAGTGAATAAGAATTTAATGTTCATTAAAATATGTCATAATCTGAGACCCAAAGCAGGT

ACCATCTGTTTCCAGTTCCTACTACATGCCTAGGTATGGAATCCATGTTCTAAAATTCTT

TTTAATTTACAACAAGTATAATTTATTTCGAAATAGCATCATCAATAACCAGTGTAATTC

TCGAATATTTTTGTTGGGATTGTTATCTGAGAAATGTGGTATTTCATTTCTTTACAA
 and

GCAAGTCATGAAATGGCTCATGCTTTTATTGCCATTTTGATGTTTTTGATGGCAAAAGTG
TTGAGAAA-AAGTCTT-TAGATTCACGTGATAAGCTGACAGAGTGAAACATCTTAAGGCT
TGAAAGGGCAAGTAGAAGTTATAATTATTGTGTAGATTCACAGTCCTTGTATTGAATTAG
TCATCTTTGCTCTCATGCTGCAGGCCATAGAGCGAGAAAAAGCTGAGAAGTTCAGAAAAC
TGCAAGATGCCAGCAGATCAGCTCAGGCCCTGGTGGAACAGATGGTGAATGGTAATTACA
CG-AGTTGATTTAGATAATCTTCTTAGGGATTTGATAAACACATAGGTTCATATTTATCA
GCTGAATTATATCAGACAAGCACTTGTTAAATACAAATTTAAATTAAAAGGTGTTTGTAT
GTTTTTATTATTCTTTTTTAATGCTAAGGAAATTATTAGGAGAAATTCAACTTTGAGT
TCATTGGAAGAAAATGGGATGTGGTAGAATATTTTATCAGTCTGTAGCAGAGAAATAAAT
TTTAATGCAAATCTG-CT-AGAATTTATCCAAATAATTTAAGAAATAAGGT-TAACAGAA
ATTGAAACATTAACAGTCAAGTATA

4. A method of assaying a sample of genomic DNA of a human subject for the presence of a mutated DMD gene, said method comprising the steps of
denaturing the DNA of said sample and digesting said DNA with one or more restriction endonucleases,
contacting said denatured, digested DNA with a human DMD probe according to any of claims 1-3 , and
detecting hybrid DNA complexes as probable indications of the presence of said DMD gene in said sample.

## Patentansprüche

1. Menschliche Duchenne-Muskeldystrophie-Sonde (DMD-Sonde), umfassend eine einstrangige Nukleinsäuresequenz, die fähig ist, an einen ersten Bereich von DNA auf einem menschlichen X-Chromosom Zu hybridisieren, wobei das menschliche X-Chromosom einen ersten Punkt, der als Löschungsbruchpunkt bei Xp21.3 bezeichnet wird, und einen zweiten Punkt aufweist, der als Translokationsbruchpunkt x;11 bezeichnet wird, wobei der erste Bereich von DNA zwischen dem ersten und zweiten Punkt positioniert ist, wobei die Nukleinsäure nicht fähig ist, an einen zweiten DNA-Bereich zwischen dem ersten Punkt und einem Terminus des X-Chromosoms zu hybridisieren, und nicht fähig ist, an einen dritten DNA-Bereich zwischen dem zweiten Punkt und dem Centromer des X-Chromosoms zu hybridisieren, wobei die zur Hybridisierung fähige Sondensequenz die gesamte oder ein Fragment der Sequenz von pERT87 ist, die unter der ATCC Zugriffsnummer 67162 hinterlegt ist.

2. Menschliche DMD-Sonde, umfassend zumindest eine der Sequenzen 
TTCTGTCTTAGTGAAGTCTCAAT-ATTTTTCAATGTGATTAAAATTCATATTATGTCACC

ATAGTTATGAAAAAAGTAAAAATATGTTTTGTATAAGTCTTAT-ATTTGATAATTTAAGC

CAATTAACAAATCCTGACAGCTGGTGTAGAATTTGAGATAAAATTCCCCTGCTGACTCAG

GCAGTGAATAAGAATTTAATGTTCATTAAAATATGTCATAATCTGAGACCCAAAGCAGGT

ACCATCTGTTTCCAGTTCCTACTACATGCCTAGGTATGGAATCCATGTTCTAAAATTCTT

TTTAATTTACAACAAGTATAATTTATTTCGAAATAGCATCATCAATAACCAGTGTAATTC

TCGAATATTTTTGTTGGGATTGTTATCTGAGAAATGTGGTATTTCATTTCTTTACAA
 und

GCAAGTCATGAAATGGCTCATGCTTTTATTGCCATTTTGATGTTTTTGATGGCAAAAGTG

TTGAGAAA-AAGTCTT-TAGATTCACGTGATAAGCTGACAGAGTGAAACATCTTAAGGCT

TGAAAGGGCAAGTAGAAGTTATAATTATTGTGTAGATTCACAGTCCTTGTATTGAATTAG

TCATCTTTGCTCTCATGCTGCAGGCCATAGAGCGAGAAAAAGCTGAGAAGTTCAGAAAAC

TGCAAGATGCCAGCAGATCAGCTCAGGCCCTGGTGGAACAGATGGTGAATGGTAATTACA

CG-AGTTGATTTAGATAATCTTCTTAGGGATTTGATAAACACATAGGTTCATATTTATCA

GCTGAATTATATCAGACAAGCACTTGTTAAATACAAATTTAAATTAAAAGGTGTTTGTAT

GTTTTTTATTATTCTTTTTTTAATGCTAAGGAAATTATTAGGAGAAATTCAACTTTGAGT

TCATTGGAAGAAAATGGGATGTGGTAGAATATTTTATCAGTCTGTAGCAGAGAAATAAAT

TTTAATGCAAATCTG-CT-AGAATTTATCCAAATAATTTAAGAAATAAGGT-TAACAGAA

ATTGAAACATTAACAGTCAAGTATA

3. Menschliche DMD-Sonde, umfassend die Sequenzen 
TTCTGTCTTAGTGAAGTCTCAAT-ATTTTTCAATGTGATTAAAATTCATATTATGTCACC
ATAGTTATGAAAAAAGTAAAAATATGTTTTGTATAAGTCTTAT-ATTTGATAATTTAAGC
CAATTAACAAATCCTGACAGCTGGTGTAGAATTTGAGATAAAATTCCCCTGCTGACTCAG
GCAGTGAATAAGAATTTAATGTTCATTAAAATATGTCATAATCTGAGACCCAAAGCAGGT
ACCATCTGTTTCCAGTTCCTACTACATGCCTAGGTATGGAATCCATGTTCTAAAATTCTT
TTTAATTTACAACAAGTATAATTTATTTCGAAATAGCATCATCAATAACCAGTGTAATTC
TCGAATATTTTTGTTGGGATTGTTATCTGAGAAATGTGGTATTTCATTTCTTTACAA
 und

GCAAGTCATGAAATGGCTCATGCTTTTATTGCCATTTTGATGTTTTTGATGGCAAAAGTG
TTGAGAAA-AAGTCTT-TAGATTCACGTGATAAGCTGACAGAGTGAAACATCTTAAGGCT
TGAAAGGGCAAGTAGAAGTTATAATTATTGTGTAGATTCACAGTCCTTGTATTGAATTAG
TCATCTTTGCTCTCATGCTGCAGGCCATAGAGCGAGAAAAAGCTGAGAAGTTCAGAAAAC
TGCAAGATGCCAGCAGATCAGCTCAGGCCCTGGTGGAACAGATGGTGAATGGTAATTACA
CG-AGTTGATTTAGATAATCTTCTTAGGGATTTGATAAACACATAGGTTCATATTTATCA
GCTGAATTATATCAGACAAGCACTTGTTAAATACAAATTTAAATTAAAAGGTGTTTGTAT
GTTTTTTATTATTCTTTTTTTAATGCTAAGGAAATTATTAGGAGAAATTCAACTTTGAGT
TCATTGGAAGAAAATGGGATGTGGTAGAATATTTTATCAGTCTGTAGCAGAGAAATAAAT
TTTAATGCAAATCTG-CT-AGAATTTATCCAAATAATTTAAGAAATAAGGT-TAACAGAA
ATTGAAACATTAACAGTCAAGTATA

4. Verfahren zum Analysieren einer Probe genomischer DNA einer menschlichen Versuchsperson auf die Gegenwart eines mutierten DND-Gens, wobei das Verfahren folgende Schritte umfaßt:
das Denaturieren der DNA der Probe und das Digerieren der DNA mit einer oder mehreren Restriktionsendonukleasen,
das In-Kontakt-Bringen der denaturierten, digerierten DNA mit einer menschlichen DMD-Sonde nach einem der Ansprüche 1 bis 3, und
das Nachweisen von Hybrid-DNA-Komplexen als wahrscheinliche Indikationen der Gegenwart des DMD-Gens in der Probe.

## Revendications

1. Sonde de myopathie de Duchenne (DMD) humaine comprenant une séquence d'acide nucléique simple brin capable de s'hybrider avec une première région d'ADN sur un chromosome X humain, ledit chromosome X humain ayant un premier point appelé point de rupture de délétion à Xp21.3 et un second point appelé point de rupture de translocation X;11, ladite première région d'ADN étant située entre ledit premier point et ledit second point, ladite séquence d'acide nucléique étant incapable de s'hybrider avec une seconde région d'ADN située entre ledit premier point et une extremité dudit chromosome X, et étant incapable de s'hybrider avec une troisième région d'ADN située entre ledit second point et le centromère dudit chromosome X, ladite séquence de sonde capable de s'hybrider étant la totalité ou un fragment de la séquence de pERT87 déposée sous le n° de référence ATCC 67162.

2. Sonde DMD humaine comprenant au moins l'une des séquences 
TTCTGTCTTAGTGAAGTCTCAAT-ATTTTTCAATGTGATTAAAATTCATATTATGTCACC
ATAGTTATGAAAAAAGTAAAAATATGTTTTGTATAAGTCTTAT-ATTTGATAATTTAAGC
CAATTAACAAATCCTGACAGCTGGTGTAGAATTTGAGATAAAATTCCCCTGCTGACTCAG
GCAGTGAATAAGAATTTAATGTTCATTAAAATATGTCATAATCTGAGACCCAAAGCAGGT
ACCATCTGTTTCCAGTTCCTACTACATGCCTAGGTATGGAATCCATGTTCTAAAATTCTT
TTTAATTTACAACAAGTATAATTTATTTCGAAATAGCATCATCAATAACCAGTGTAATTC
TCGAATATTTTTGTTGGGATTGTTATCTGAGAAATGTGGTATTTCATTTCTTTACAA
 et

GCAAGTCATGAAATGGCTCATGCTTTTATTGCCATTTTGATGTTTTTGATGGCAAAAGTG
TTGAGAAA-AAGTCTT-TAGATTCACGTGATAAGCTGACAGAGTGAAACATCTTAAGGCT
TGAAAGGGCAAGTAGAAGTTATAATTATTGTGTAGATTCACAGTCCTTGTATTGAATTAG
TCATCTTTGCTCTCATGCTGCAGGCCATAGAGCGAGAAAAAGCTGAGAAGTTCAGAAAAC
TGCAAGATGCCAGCAGATCAGCTCAGGCCCTGGTGGAACAGATGGTGAATGGTAATTACA
CG-AGTTGATTTAGATAATCTTCTTAGGGATTTGATAAACACATAGGTTCATATTTATCA
GCTGAATTATATCAGACAAGCACTTGTTAAATACAAATTTAAATTAAAGGTGTTTGTAT
GTTTTTTATTATTCTTTTTTTAATGCTAAGGAAATTATTAGGAGAAATTCAACTTTGAGT

TCATTGGAAGAAAATGGGATGTGGTAGAATATTTTATCAGTCTGTAGCAGAGAAATAAAT
TTTAATGCAAATCTG-CT-AGAATTTATCCAAATAATTTAAGAAATAAGGT-TAACAGAA
ATTGAAACATTAACAGTCAAGTATA

3. Sonde DMD humaine comprenant les séquences

TTCTGTCTTAGTGAAGTCTCAAT-ATTTTTCAATGTGATTAAAATTCATATTATGTCACC

ATAGTTATGAAAAAAGTAAAAATATGTTTTGTATAAGTCTTAT-ATTTGATAATTTAAGC

CAATTAACAAATCCTGACAGCTGGTGTAGAATTTGAGATAAAATTCCCCTGCTGACTCAG

GCAGTGAATAAGAATTTAATGTTCATTAAAATATGTCATAATCTGAGACCCAAAGCAGGT

ACCATCTGTTTCCAGTTCCTACTACATGCCTAGGTATGGAATCCATGTTCTAAAATTCTT

TTTAATTTACAACAAGTATAATTTATTCGAAATAGCATCATCAATAACCAGTGTAATTC

TCGAATATTTTTGTTGGGATTGTTATCTGAGAAATGTGGTATTTCATTTCTTTACAA et

GCAAGTCATGAAATGGCTCATGCTTTTATTGCCATTTTGATGTTTTTGATGGCAAAAGTG

TTGAGAAA-AAGTCTT-TAGATTCACGTGATAAGCTGACAGAGTGAAACATCTTAAGGCT

TGAAAGGGCAAGTAGAAGTTATAATTATTGTGTAGATTCACAGTCCTTGTATTGAATTAG

TCATCTTTGCTCTCATGCTGCAGGCCATAGAGCGAGAAAAAGCTGAGAAGTTCAGAAAAC

TGCAAGATGCCAGCAGATCAGCTCAGGCCCTGGTGGAACAGATGGTGAATGGTAATTACA

CG-AGTTGATTTAGATAATCTTCTTAGGGATTTGATAAACACATAGGTTCATATTTATCA

GCTGAATTATATCAGACAAGCACTTGTTAAATACAAATTTAAATTAAAGGTGTTTGTAT

GTTTTTTATTATTCTTTTTTAATGCTAAGGAAATTATTAGGAGAAATTCAACTTTGAGT

TCATTGGAAGAAAATGGGATGTGGTAGAATATTTTATCAGTCTGTAGCAGAGAAATAAAT

TTTAATGCAAATCTG-CT-AGAATTTATCCAAATAATTTAAGAAATAAGGT-TAACAGAA

ATTGAAACATTAACAGTCAAGTATA

4. Procédé pour tester un échantillon d'ADN génomique d'un sujet humain en ce qui concerne la présence d'un gène DMD muté, ledit procédé comprenant les étapes consistant à
dénaturer l'ADN dudit échantillon et digérer ledit ADN avec une ou plusieurs endonucléases de restriction,
mettre en contact ledit ADN dénaturé et digéré avec une sonde DMD humaine selon l'une quelconque des revendications 1 à 3, et
détecter les complexes d'ADN hybride en tant qu'indications probables de la présence dudit gène DMD dans ledit échantillon.
